# EUROPÄISCHE PATENTSCHRIFT

(11) **EP 1 289 912 B1**
(45) Veröffentlichungstag und Bekanntmachung des Hinweises auf die Patenterteilung: **30.08.2006**
(21) Anmeldenummer: 01943427.3
(22) Anmeldetag: 22.05.2001
(51) Int. Cl.: C07C 11/06

(54) **VERFAHREN ZUM ERZEUGEN VON PROPYLEN AUS METHANOL**
METHOD FOR PRODUCING PROPYLENE FROM METHANOL
PROCEDE DE PRODUCTION DE PROPYLENE A PARTIR DE METHANOL

(30) Priorität: 31.05.2000 DE 10027159; 06.04.2001 DE 10117248
(43) Veröffentlichungstag der Anmeldung: 12.03.2003
(73) Patentinhaber: MG Technologies AG, 60325 Frankfurt am Main (DE)
(72) Erfinder: HACK, Markus, 61184 Karben (DE); KOSS, Ulrich, 64289 Darmstadt (DE); KÖNIG, Peter, 61348 Bad Homburg (DE); ROTHAEMEL, Martin, 60437 Frankfurt am Main (DE); HOLTMANN, Hans-Dieter, 59199 Boenen (DE)
(86) Internationale Anmeldenummer: PCT/EP2001/005855
(87) Internationale Veröffentlichungsnummer: WO 2001/092190

(56) Entgegenhaltungen:
- EP-A- 0 448 000
- DE-A- 19 723 363

## Beschreibung

Verfahren zum Erzeugen von Propylen aus Methanol, wobei man Methanoldampf an einem ersten Katalysator zu einem Dimethylether (DME) enthaltenden ersten Dampfgemisch umsetzt und aus dem ersten Dampfgemisch an einem formselektiven Zeolith-Katalysator vom Pentasiltyp ein Propylen enthaltendes Produktgemisch erzeugt.

Verfahren dieser Art sind bekannt und zum Beispiel in EP 0448 000 B1 und DE 197 23 363 A1 beschrieben. Der formselektive Zeolith-Katalysator ist hierbei in einem Röhrenreaktor angeordnet und wird indirekt gekühlt, um entstehende Wärme abzuführen.

Der Erfindung liegt die Aufgabe zugrunde, einen möglichst hohen Anteil Propylen im Produktgemisch zu erreichen. Gleichzeitig soll auf den aufwendigen Röhrenreaktor verzichtet werden können, um das Verfahren möglichst kostengünstig zu gestalten.

Die Aufgabe wird erfindungsgemäß beim eingangsgenannten Verfahren dadurch gelöst, dass der Zeolith-Katalysator als Schüttung in mindestens zwei in Serie geschalteten Schachtreaktoren ohne Einrichtungen zur indirekten Kühlung angeordnet ist, dass man einen ersten Teilstrom des DME enthaltenden ersten Dampfgemisches unter Zusatz von Wasserdampf in den ersten Schachtreaktor führt, aus dem ersten Schachtreaktor ein erstes Zwischenproduktgemisch abzieht und es dem zweiten Schachtreaktor aufgibt, wobei man dem zweiten Schachtreaktor auch einen zweiten Teilstrom des DME enthaltenden ersten Dampfgemisches zuführt, dass man aus dem letzten der in Serie geschalteten Schachtreaktoren Produktgemisch abzieht, kühlt, eine propanreiche Fraktion abtrennt und Reststoffe erhält, die teilweise gasförmig sind und C₃₊₋Kohlenwasserstoffe enthalten, und dass man mindestens einen Teil der Reststoffe in mindestens einen der Schachtreaktoren zurückführt. Üblicherweise wird der formselektive Zeolith-Katalysator in höchstens vier oder fünf in Serie geschalteten Schachtreaktoren als Schüttung angeordnet sein. Die Abtrennung der propanreichen Fraktion kann in an sich bekannter Weise z.B. destillativ oder adsorptiv erfolgen.

Der erste Katalysator, an welchem das Methanol zunächst teilweise umgewandelt wird, ist üblicherweise ebenfalls als Schüttung in einem Schachtreaktor enthalten, hierbei kann es sich in an sich bekannter Weise um einen Al₂O₃-Katalysator handeln. Einzelheiten zum ersten Katalysator sind aus EP 0 448 000 B1 und DE 197 23 363 A1 bekannt. In diesen Veröffentlichungen wird auch der formselektive Zeolith-Katalysator beschrieben, der im erfindungsgemäßen Verfahren verwendet werden kann. Es handelt sich hierbei um einen Protonen enthaltenden Katalysator vom Pentasil-Typ mit einem Alkaligehalt von weniger als 380 ppm und vorzugsweise weniger als 200 ppm. Dieser Katalysator weist einen ZnO-Gehalt von weniger als 0,1 Gew.-%, einen CdO-Gehalt von weniger als 0,1 Gew.-%, eine BET-Oberfläche von 300 bis 600 m²/g und ein Porenvolumen (nach der Quecksilberporosimetrie bestimmt) von 0,3 bis 0,8 m³/g auf. Üblicherweise liegt der Druck im Bereich dieses Katalysators bei höchstens 0,9 bar und vorzugsweise im Bereich von 0,2 bis 0;7 bar.

Dem ersten Schachtreaktor, der den Zeolith-Katalysator enthält, gibt man ein Gemisch auf, welches üblicherweise zu 10 bis 40 Vol. % (trocken gerechnet) aus DME besteht. Gleichzeitig sorgt man für einen ausreichenden Wasserdampf-Gehalt in dem Gemisch, wobei der H₂O-Anteil im Gemisch im Bereich von 40 bis 80 Vol.-% liegt. Für nachfolgende Schachtreaktoren gelten bezüglich des H₂O-Gehalts im in den jeweiligen Reaktor eintretenden Gemisch dieselben Bedingungen. Üblicherweise führt man jedem Schachtreaktor mindestens 10% des vom ersten Katalysator kommenden ersten Dampfgemisches zu.

Die Temperaturen am Eingang der Schachtreaktoren, in denen der Zeolith-Katalysator enthalten ist, liegen im Bereich von 350 bis 500 °C und zumeist 380 bis 480 °C. Durch die Verwendung von Schachtreaktoren ohne Einrichtungen zur indirekten Kühlung vereinfachen sich deren Herstellung und Betrieb beträchtlich. Man sorgt dafür, dass die Temperatur am Ausgang eines oder mehrerer der Schachtreaktoren um 50 bis 100 °C höher ist als am Eingang des jeweiligen Schachtreaktors.

Eine vorteilhafte Weiterbildung des Verfahrens besteht darin, dass man das aus dem letzten Schachtreaktor abgezogene, Wasserdampf enthaltende Produktgemisch auf Temperaturen im Bereich von 100 bis 250 °C kühlt, auf einen Druck im Bereich von 3 bis 15 bar verdichtet und ein verdichtetes Produktgemisch erzeugt, dessen H₂O-Gehalt zu höchstens 30 Gew. % verflüssigt ist. Man führt das verdichtete Produktgemisch durch mindestens einen indirekten Wärmeaustauscher und kühlt es darin gegen eine Wasserphase ab. Aus dem Wärmeaustauscher zieht man ein Kondensat enthaltendes, gekühltes Produktgemisch ab, dessen H₂O-Gehalt zu mindestens 80 Gew. % verflüssigt ist und dessen Temperatur um 20 bis 150 °C niedriger als am Eintritt in den Wärmeaustauscher ist Auf diese Weise wird Kondensationswärme auf die Wasserphase übertragen. Aus dem gekühlten kondensathaltigen Produktgemisch wird eine Wasserphase abgetrennt und die Wasserphase wird zurück in den indirekten Wärmeaustauscher geleitet, wo die Wasserphase ganz oder weitgehend verdampft. Der erzeugte Wasserdampf wird mindestens teilweise in den ersten Schachtreaktor geleitet

Ausgestaltungsmöglichkeiten des Verfahrens werden mit Hilfe der Zeichnung erläutert.
Fig. 1 zeigt das Fließschema einer ersten Verfahrensvariante und
Fig. 2 zeigt das Fließschema einer zweiten Verfahrensvariante.

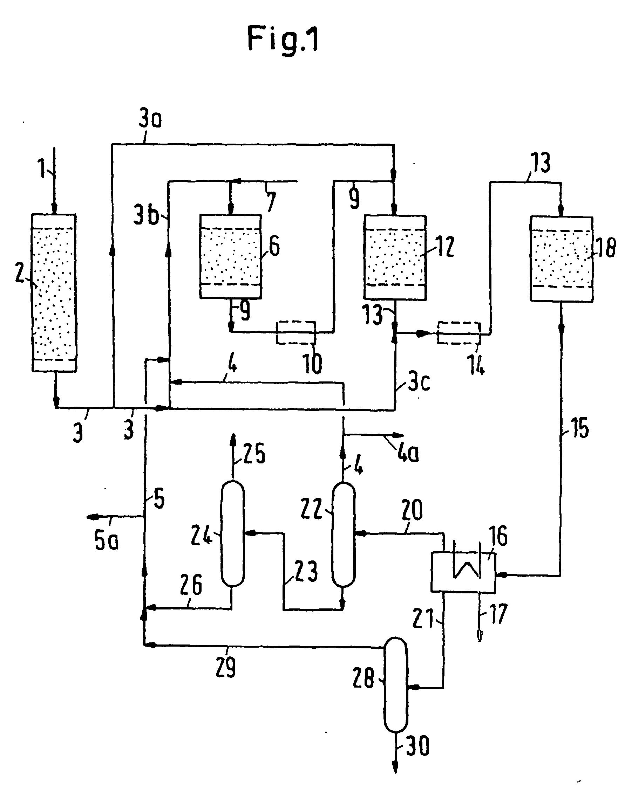

Gemäß Fig. 1 wird der umzusetzende Methanoldampf, der Temperaturen üblicherweise im Bereich von 200 bis 350 °C aufweist in der Leitung (1) herangeführt und durch die Schüttung des ersten Katalysators (2) geleitet. Am ersten Katalysator, der zum Beispiel aus kömigem Al₂O₃ besteht, findet eine erste exotherme Umwandlung statt und man erhält in der Leitung (3) ein erstes Dampfgemisch, welches üblicherweise mindestens 50 Vol. % DME und daneben noch Methanol und Wasserdampf enthält. Die Temperatur der Leitung (3) liegt im Bereich von 350 bis 450 °C. Das Dampfgemisch der Leitung (3) wird auf die Leitungen (3a), (3b) und (3c) aufgeteilt. Der Teilstrom in der Leitung (3b) wird hier auch als "erster Teilstrom" und der in der Leitung (3a) als "zweiter Teilstrom" bezeichnet.

Zusammen mit Reststoffen aus den Leitungen (4) und (5), die gekühlt herangeführt werden, gibt man das Gemisch der Leitung (3b) in den ersten Schachtreaktor (6), der eine Schüttung des formselektiven Zeolith-Katalysators enthält. Wasserdampf wird in der Leitung (7) herangeführt. Man sorgt dafür, dass die Temperatur des Gemisches, welches im Reaktor (6) in die Katalysatorschüttung eintritt, im Bereich von 350 bis 500 °C und vorzugsweise 380 bis 480 °C liegt. Am Katalysator im Reaktor (6) laufen exotherme Umwandlungsreaktionen ab und man erhält in der Leitung (9) ein erstes Zwischenproduktgemisch mit Temperaturen im Bereich von 400 bis 600 °C. Bei Bedarf kann dieses Gemisch durch einen indirekten Kühler (10) geführt werden, der nicht in allen Fällen erforderlich und in der Zeichnung gestrichelt angedeutet ist.

Dem Gemisch der Leitung (9) gibt man den Teilstrom aus der Leitung (3a) zu, welcher hier auch als "zweiter Teilstrom" bezeichnet wird. Die weitere Umsetzung erfolgt im zweiten Schachtreaktor (12), der ebenfalls eine Schüttung des formselektiven Zeolith-Katalysators enthält. Die Verfahrens-Bedingungen im Reaktor (12) sind in etwa die gleichen wie im Reaktor (6), und dies gilt auch für den dritten Schachtreaktor (18). Aus dem Reaktor (12) erhält man in der Leitung (13) ein zweites Zwischenproduktgemisch, welchemman den dritten Teilstrom aus der Leitung (3c) zugibt. Auch hier kann das Gemisch in der Leitung (13) bei Bedarf durch einen indirekten Kühler (10) geführt werden.

Das Gemisch der Leitung (13) wird durch den dritten Schachtreaktor (18) geführt, der im vorliegenden Fall der letzte der in Serie geschalteten Schachtreaktoren ist, welche einen Zeolith-Katalysator als Schüttung enthalten. Das in der Leitung (15) abgezogene Produktgemisch weist üblicherweise einen Propylengehalt, trocken gerechnet, von 40 bis 60 Vol. % auf und enthält daneben noch zusätzlich andere Substanzen, die hier auch als Reststoffe bezeichnet werden.

Das Gemisch der Leitung (15) wird zunächst einer Kühlung (16) unterzogen, wobei man ein wasserreiches Kondensat erhält, welches in der Leitung (17) abgeführt wird. Gas- und dampfförmige Stoffe zieht man in der Leitung (20) ab und ein Flüssigkeitsgemisch wird in der Leitung (21) abgeführt. Die Gase und Dämpfe, in denen auch das gewünschte Propylen enthalten ist, gibt man einer ersten Kolonne (22) auf, trennt Gase ab und führt sie in der Leitung (4) zurück, wie beschrieben. Das Sumpfprodukt der Kolonne (22) gelangt durch die Leitung (23) in eine zweite Kolonne (24), von deren Kopf man durch die Leitung (25) eine propylenreiche Fraktion mit einem Propylengehalt von üblicherweise mindestens 80 Vol. % abzieht. Das die Kolonne (24) in der Leitung (26) verlassende Sumpfprodukt (zumeist C₄₊₋Kohlenwasserstoffe) wird in der Leitung (5) zurückgeführt. Die Reststoffe der Leitungen (4) und (5) können auch teilweise in die Gemische der Leitung (3a) und/oder der Leitung (3c) gegeben werden. Überschüsse werden durch die Leitungen (4a) und (5a) entfernt.

Das Flüssigkeitsgemisch der Leitung (21) gelangt in die dritte Kolonne (28), aus welcher man eine leichte C₅₊-Fraktion abtrennt und in der Leitung (29) und durch die Leitung (5) zurückführt. Die schweren Bestandteile, üblicherweise Benzinkohlenwasserstoffe, zieht man durch die Leitung (30) ab und entfernt sie aus dem Verfahren.

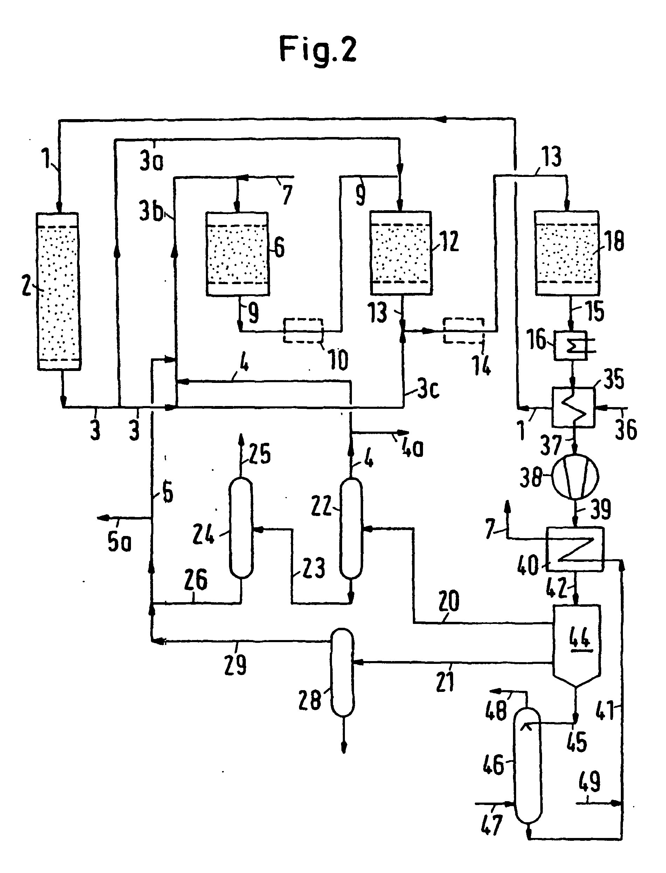

Beim Verfahren der Fig. 2 nutzt man den Wärmeinhalt des aus dem letzten Schachtreaktor (18) in der Leitung (15) kommenden Produktgemisches, welches üblicherweise Temperaturen im Bereich von 400 - 600°C aufweist. Zunächst gibt das Produktgemisch einen Teil seiner Wärme im Wärmeaustauscher (16) und dann im Vorwärmer (35) ab, dem man durch die Leitung (36) Methanol flüssig und/oder dampfförmig zuführt. Aus dem Vorwärmer (35) zieht man Methanoldampf mit Temperaturen im Bereich von 200 - 350°C in der Leitung (1) ab und leitet ihn durch die Schüttung des ersten Katalysators (2). Mit Temperaturen von 100 - 250°C strömt das Produktgemisch, welches auch Wasserdampf enthält, durch die Leitung (37) zu einem Kompressor (38) und weist an dessen Ausgang in der Leitung (39) einen Druck von 3-15 bar und aus ökonomischen Gründen zumeist höchstens 10 bar auf, die Temperatur liegt im Bereich von 130 - 250°C. Der Wasserdampf des Gemisches in der Leitung (39) ist noch nicht oder kaum kondensiert, höchstens 30 Gew.-% und vorzugsweise höchstens 10 Gew.-% des H₂O-Gehalts sind bereits verflüssigt.

Im indirekten Wärmeaustauscher (40) kühlt das Produktgemisch der Leitung (39) weiter ab, wobei eine Wasserphase als Kühlmedium dient, die in der Leitung (41) herangeführt wird. Dabei verdampft die Wasserphase ganz oder weitgehend und der erzeugte Wasserdampf wird in der Leitung (7) abgeführt, die Temperatur liegt zwischen 100 und 200°C und der Druck zwischen 0,1 und 10 bar. Diese Leitung (7) mündet in die Leitung (3 b), was der besseren Übersichtlichkeit wegen nicht vollständig gezeichnet wurde.

Das aus dem Wärmeaustauscher (40) in der Leitung (42) kommende Produktgemisch ist teilweise kondensiert und mindestens 80 Gew. -% des H₂O-Gehalts sind verflüssigt. Die Temperaturen liegen in der Leitung (42) um 20 - 150°C und zumeist 30 - 120°C niedriger als in der Leitung (39) und auch der Druck ist um 0,1 - 10 bar gesunken. Zum Trennen gibt man das Produktgemisch der Leitung (42) einem Separator (44) auf, aus dem man in der Leitung (45) eine Wasserphase und in der Leitung (21) ein Benzinkohlenwasserstoffe enthaltendes Flüssigkeitsgemisch abzieht. Gase und Dämpfe, die auch das gewünschte Propylen enthalten, ziehen in der Leitung (20) ab. Die Wasserphase der Leitung (45) kann durch die Leitung (41) zurück zum Wärmeaustauscher (40) geführt werden. Im vorliegenden Fall gibt man sie dem Stripper (46) auf, um mittels Strippgas (z.B. Stickstoff) aus der Leitung (47) leichtsiedende Kohlenwasserstoffe (z.B. C₂-Kohlenwasserstoffe) über die Leitung (48) zu entfernen. Die gestrippte Wasserphase gelangt in der Leitung (41) zurück zum Wärmeaustauscher (40), Frischwasser wird in der Leitung (49) herangeführt. Im übrigen gelten die zu Fig. 1 gegebenen Erläuterungen.

### Beispiel 1:

Man arbeitet mit einer der Fig. 1 der Zeichnung entsprechenden Anlage, die nachfolgenden Daten sind teilweise berechnet.

Dem ersten Katalysator (2), der aus körnigem Al₂O₃ besteht, führt man auf 280°C erhitzten Methanoldampf zu und man erhält in der Leitung (3) ein Dampfgemisch von 382°C, welches aus 32 Vol. -% Methanol, 34 Vol. -% DME und 34 Vol. -% Wasserdampf besteht. Dieses Dampfgemisch wird auf die Leitungen (3a), (3b) und (3c) im Verhältnis 1:1,3:1,8 aufgeteilt. Das Gewichtsverhältnis des Dampfgemisches in der Leitung (3b) zum durch die Leitung (7) herangeführten Wasserdampf beträgt 1:4. Das in den ersten Schachtreaktor (6) eintretende Gemisch hat eine Temperatur von 435°C und einen Druck von 1,8 bar. Der formselektive Zeolith-Katalysator vom Pentasil-Typ, der in den Schachtreaktoren (6), (12) und (18) verwendet wird, hat einen Alkaligehalt von 100 ppm, einen Gehalt an ZnO + CdO von 0,05 Gew. -%, eine BET-Oberfläche von 460 m³/g und ein Porenvolumen von 0,4 m³/g. In allen drei Schachtreaktoren arbeitet man mit einer Raumgeschwindigkeit von 1 kg Methanol -

Äquivalent pro kg Katalysator und pro Stunde (1 Mol DME= 2 Mol Methanol - Äquivalent).

Das Gemisch in der Leitung (9) hat eine Temperatur von 495°C, die Temperatur im Eintritt zum Schachtreaktor (12) liegt bei 440°C, die gleiche Eintrittstemperatur hat der Schachtreaktor (18). Aus dem Produktgemisch der Leitung (15) trennt man Prozesswasser (17) durch Kühlung (16) ab und führt die gasförmigen Bestandteile durch die Leitung (20) zur Kolonne (22). Die weitere Arbeitsweise ist wie zusammen mit der Zeichnung beschrieben. Jeweils 10% der in den Leitungen (4) und (5) strömenden Mengen werden durch die Leitungen (4a) und (5a) abgezogen.

Durch die Leitung (29) zieht man 80% der umsetzbaren C₅- bis C₈ - Olefine ab und in der Leitung (30) erhält man Naphtha. Das Gasgemisch der Leitung (4) besteht zu 40 Vol. -% aus Ethylen, zu 30 Vol. -% aus Methan und im übrigen aus Ethan, H2 und CO. Das Gemisch der Leitung (26) besteht zu 50 Vol. -% aus Buten und zu 30 Vol. -% aus Butan, im übrigen überwiegend aus Penten und Pentan. 58 Vol. -% des Gemisches der Leitung (29) besteht aus C₅- bis C₈ - Olefinen und im übrigen aus Paraffin-Kohlenwasserstoffen.

70 Mol. -% des eingesetzten Methanols ergibt den Produktstrom der Leitung (25), er besteht zu 97 Vol. -% aus Propylen, 26 Mol. -% des eingesetzten Methanols werden als Naphtha durch die Leitung (5a) abgeführt und 4 Mol. -% ergibt Heizgas Leitung (4a). Nach einer Anfahrphase kann der in der Leitung (7) zugeführte Wasserdampf um ein Viertel reduziert werden.

### Beispiel 2:

Im wesentlichen wird wie im Beispiel 1 gearbeitet, jedoch wird das Produktgemisch der Leitung (15) wie in Fig. 2 beschrieben weiter behandelt. Auf den Stripper (46) wird verzichtet, die Leitungen (45) und (41) werden miteinander verbunden, das Zusatzwasser der Leitung (49) entfällt. Wichtige Daten ergeben sich aus der nachfolgenden Tabelle:

| Bezugsziffer | 37 | 39 | 42 | 7 | 20 | 21 |
|---|---|---|---|---|---|---|
| Temperatur (°C) | 180 | 185 | 120 | 113 | 120 | 120 |
| Druck (bar) | 1,3 | 5,5 | 4,5 | 1,6 | 2,0 | 2,0 |

## Patentansprüche

1. Verfahren zum Erzeugen von Propylen aus Methanol, wobei man Methanoldampf an einem ersten Katalysator zu einem Dimethylether (DME) enthaltenden ersten Dampfgemisch umsetzt und aus dem ersten Dampfgemisch an einem formselektiven Zeolith-Katalysator vom Pentasiltyp ein Propylen enthaltendes Produktgemisch erzeugt, **dadurch gekennzeichnet, dass** der Zeolith-Katalysator als Schüttung in mindestens zwei in Serie geschalteten Schachtreaktoren ohne Einrichtungen zur indirekten Kühlung angeordnet ist, dass man einen ersten Teilstrom des DME enthaltenden ersten Dampfgemisches unter Zusatz von Wasserdampf in den ersten Schachtreaktor führt, aus dem ersten Schachtreaktor ein erstes Zwischenproduktgemisch abzieht und es dem zweiten Schachtreaktor aufgibt, wobei man dem zweiten Schachtreaktor auch einen zweiten Teilstrom des DME enthaltenden ersten Dampfgemisches zuführt, dass man aus dem letzten der in Serie geschalteten Schachtreaktoren ein Produktgemisch abzieht, kühlt, eine propanreiche Fraktion abtrennt und Reststoffe erhält, die teilweise gasförmigen sind und C₃₊₋Kohlenwasserstoffe enthalten und dass man mindestens einen Teil der Reststoffe in mindestens einen der Schachtreaktoren zurückführt.

2. Verfahren nach Anspruch 1, **dadurch gekennzeichnet, dass** das Gemisch am Eingang zum ersten Schachtreaktor zu 10 bis 40 Vol. % DME besteht.

3. Verfahren nach Anspruch 1 oder 2, **dadurch gekennzeichnet, dass** man das aus dem letzten Schachtreaktor abgezogene, Wasserdampf enthaltende Produktgemisch auf Temperaturen im Bereich von 100 bis 250 °C kühlt, auf einen Druck im Bereich von 3 bis 15 bar verdichtet und ein verdichtetes Produktgemisch erzeugt, dessen H₂O-Gehalt zu höchstens 30 Gew. % verflüssigt ist, dass man das verdichtete Produktgemisch durch mindestens einen indirekten Wärmeaustauscher führt und es darin gegen eine Wasserphase kühlt, dass man aus dem Wärmeaustauscher ein Kondensat enthaltendes, gekühltes Produktgemisch abzieht, dessen H₂O-Gehalt zu mindestens 80 Gew. % verflüssigt ist und dessen Temperatur um 20 bis 150 °C niedriger als am Eintritt in den Wärmeaustauscher ist, dass man aus dem kondensathaltigen Produktgemisch eine Wasserphase abtrennt und die Wasserphase in den indirekten Wärmeaustauscher leitet und dass man die Wasserphase im Wärmeaustauscher ganz oder weitgehend verdampft und den erzeugten Wasserdampf mindestens teilweise in den ersten Schachtreaktor leitet.

4. Verfahren nach Anspruch 3, **dadurch gekennzeichnet, dass** man aus der Wasserphase leicht flüchtige Kohlenwasserstoffe entfernt, bevor man die Wasserphase in den indirekten Wärmeaustauscher leitet.

5. Verfahren nach Anspruch 1 oder einem der folgenden, **dadurch gekennzeichnet, dass** drei Schachtrektoren in Serie geschaltet sind, wobei man dem dritten Schachtreaktor ein vom zweiten Schachtreaktor kommendes zweites Zwischenproduktgemisch und einen dritten Teilstrom des DME enthaltenden ersten Dampfgemisches zuführt und Produktgemisch aus dem dritten Schachtreaktor abzieht.

6. Verfahren nach einem der Ansprüche 1 bis 5, **dadurch gekennzeichnet, dass** die Temperaturen am Eingang der Schachtreaktoren im Bereich von 350 bis 500 °C liegen.

7. Verfahren nach Anspruch 1 oder einem der folgenden, **dadurch gekennzeichnet, dass** die Temperatur bei einem oder mehreren der Schachreaktoren am Ausgang um 30 bis 100 °C höher ist als am Eingang.

8. Verfahren nach Anspruch 1 oder einem der folgenden, **dadurch gekennzeichnet, dass** das vom letzten Schachtreaktor abgeführte Produktgemisch eine Propylengehalt, trocken gerechnet, von 20 bis 50 Vol. % aufweist.

## Claims

1. A process for producing propylene from methanol, wherein methanol vapor is reacted on a first catalyst to obtain a first vapor mixture containing dimethyl ether (DME); and from the first vapor mixture a product mixture containing propylene is produced on a form-selective zeolite catalyst of the pentasil type, **characterized in that** the zeolite catalyst is arranged as bed in at least two series-connected shaft reactors without any means for indirect cooling, that a first partial stream of the first vapor mixture containing DME is introduced into the first shaft reactor by adding water vapor, from the first shaft reactor a first intermediate product mixture is withdrawn and charged into the second shaft reactor, wherein a second partial stream of the first vapor mixture containing DME is also supplied to the second shaft reactor, that from the last one of the series-connected shaft reactors a product mixture is withdrawn, cooled, a fraction rich in propane is separated, and residual substances are obtained, which are partly gaseous and contain C₃₊ hydrocarbons, and that at least a part of the residual substances is recirculated into at least one of the shaft reactors.

2. The process as claimed in claim 1, **characterized in that** the mixture at the inlet of the first shaft reactor consists of 10 to 40 vol-% DME.

3. The process as claimed in claim 1 or 2, **characterized in that** the product mixture withdrawn from the last shaft reactor, which contains water vapor, is cooled to temperatures in the range from 100 to 250°C, is compressed to a pressure in the range from 3 to 15 bar, and a compressed product mixture is produced, whose H₂O content is liquefied for 30 wt-% at most, that the compressed product mixture is passed through at least one indirect heat exchanger and is cooled therein against a water phase, that from the heat exchanger a cooled product mixture containing condensate is withdrawn, whose H₂O content is liquefied for at least 80 wt-% and whose temperature is by 20 to 150°C lower than at the inlet of the heat exchanger, that from the product mixture containing condensate a water phase is separated and the water phase is introduced into the indirect heat exchanger, and that the water phase is largely or completely evaporated in the heat exchanger and the water vapor produced is at least partly introduced into the first shaft reactor.

4. The process as claimed in claim 3, **characterized in that** highly volatile hydrocarbons are removed from the water phase before the water phase is introduced into the indirect heat exchanger.

5. The process as claimed in claim 1 or any of the preceding claims, **characterized in that** three shaft reactors are connected in series, wherein a second intermediate product mixture coming from the second shaft reactor and a third partial stream of the first vapor mixture containing DME are supplied to the third shaft reactor and product mixture is withdrawn from the third shaft reactor.

6. The process as claimed in any of claims 1 to 5, **characterized in that** the temperatures at the inlet of the shaft reactors lie in the range from 350 to 500°C.

7. The process as claimed in claim 1 or any of the preceding claims, **characterized in that** the temperature at the outlet of one or more of the shaft reactors is by 30 to 100°C higher than at the inlet.

8. The process as claimed in claim 1 or any of the preceding claims, **characterized in that** the product mixture discharged from the last shaft reactor has a propylene content of 20 to 50 vol-%, calculated dry.

## Revendications

1. Procédé de production de propylène à partir de méthanol, dans lequel on convertit de la vapeur de méthanol sur un premier catalyseur en un premier mélange de vapeurs contenant de l'éther diméthylique (DME) et on produit un mélange de produits contenant du propylène à partir d'un premier mélange de vapeurs sur un catalyseur de zéolithe à sélection de forme du type pentasil, **caractérisé en ce que** le catalyseur de zéolithe est disposé en vrac dans au moins deux réacteurs en puits, montés en série sans dispositif pour un refroidissement indirect, **en ce que** l'on introduit un premier courant partiel du premier mélange de vapeurs contenant DME dans le premier réacteur en puits en ajoutant de la vapeur d'eau, on soutire d'un premier réacteur en puits, un premier mélange de produits intermédiaires et on le fait passer dans le deuxième réacteur en puits, en introduisant également un deuxième courant partiel du premier mélange de vapeurs contenant DME dans le deuxième réacteur en puits, **en ce que** l'on soutire un mélange de produits à partir du dernier des réacteurs en puits montés en série, on le refroidit, on en sépare une fraction riche en propane, et on obtient des matières résiduelles qui sont partiellement gazeuses et contiennent des hydrocarbures en C₃₊ et **en ce que** l'on recycle au moins une partie des matières résiduelles dans au moins l'un des réacteurs en puits.

2. Procédé selon la revendication 1, **caractérisé en ce que** le mélange se compose de 10 à 40% en volume de DME à l'entrée du premier réacteur en puits.

3. Procédé selon la revendication 1 ou 2, **caractérisé en ce que** l'on refroidit le mélange de produits contenant de la vapeur d'eau, soutiré du dernier réacteur en puits, à des températures dans la plage de 100 à 250°C et on le comprime à une pression dans la plage de 3 à 15 bars, et on produit un mélange de produits comprimé dont la teneur en H₂O est condensée à raison de 30% en poids au maximum, **en ce que** l'on fait passer le mélange de produits comprimé à travers au moins un échangeur de chaleur indirect et qu'il s'y refroidit contre une phase aqueuse, **en ce que** l'on soutire de l'échangeur de chaleur, un mélange de produits refroidi contenant un condensat, dont la teneur en H₂O est condensée à raison d'au moins 80% en poids et dont la température est inférieure de 20 à 150°C à celle à l'entrée dans l'échangeur de chaleur, **en ce que** l'on sépare une phase aqueuse à partir du mélange de produits contenant le condensat et que l'on conduit la phase aqueuse dans l'échangeur de chaleur indirect et **en ce que** l'on évapore totalement ou en grande partie la phase aqueuse dans l'échangeur de chaleur que l'on conduit la vapeur d'eau produite au moins partiellement dans le premier réacteur en puits.

4. Procédé selon la revendication 3, **caractérisé en ce que** l'on retire les hydrocarbures facilement volatils de la phase aqueuse, avant de conduire la phase aqueuse dans l'échangeur de chaleur indirect.

5. Procédé selon la revendication 1 ou l'une des suivantes, **caractérisé en ce que** les trois réacteurs en puits sont montés en série, dans lequel l'on introduit dans le troisième réacteur en puits, un deuxième mélange de produits intermédiaire provenant du deuxième réacteur en puits et un troisième courant partiel du premier mélange de vapeurs contenant DME et on soutire le mélange de produits à partir du troisième réacteur en puits.

6. Procédé selon l'une quelconque des revendications 1 à 5, **caractérisé en ce que** les températures à l'entrée des réacteurs en puits se trouvent dans la plage de 350 à 500°C.

7. Procédé selon la revendication 1 ou l'une des suivantes, **caractérisé en ce que** la température à la sortie pour l'un ou plusieurs des réacteurs en puits est supérieure de 30 à 100°C à celle à l'entrée.

8. Procédé selon la revendication 1 ou l'une des suivantes, **caractérisé en ce que** le mélange de produits évacué du dernier réacteur en puits présente une teneur en propylène, calculée à l'état sec, de 20 à 50% en volume.
